# EUROPÄISCHE PATENTSCHRIFT

(11) **EP 0 994 181 B1**
(45) Veröffentlichungstag und Bekanntmachung des Hinweises auf die Patenterteilung: **22.01.2003**
(21) Anmeldenummer: 99118043.1
(22) Anmeldetag: 22.09.1999
(51) Int. Cl.: C12M 1/04, C12M 3/06

(54) **Verfahren und Vorrichtung zur Erfassung der Interaktionen des Stoffwechsels von Aeroben und Anaeroben Zellsystemen**
Procédé et appareil pour la détection des interactions des métabolismes de cellules aérobiques et anaérobiques
Process and apparatus for the detection of the interactions of the metabolisms of aerobic and anaerobic cellsystems

(30) Priorität: 29.09.1998 DE 19844708
(43) Veröffentlichungstag der Anmeldung: 19.04.2000
(73) Patentinhaber: GSF-Forschungszentrum für Umwelt und Gesundheit GmbH, 85764 Oberschleissheim (DE)
(72) Erfinder: Laube, Britta, 85452 Moosinning (DE); Schwarz, Leslie R., Dr., 85354 Freising (DE); Wiebel, Friedrich J., Prof. Dr., 85386 Eching (DE)
(74) Vertreter: Reinhard - Skuhra - Weise & Partner

(56) Entgegenhaltungen:
- EP-A- 0 049 954
- EP-A- 0 444 585
- EP-A- 0 620 274
- EP-A- 0 725 134
- DE-C- 839 245
- GB-A- 2 268 187
- US-A- 3 919 053

## Beschreibung

Die Erfindung betrifft ein Verfahren und eine Vorrichtung zur Erfassung der Interaktionen des Stoffwechsels von aeroben und anaeroben Zellsystemen. Ein In vitro-System wird dabei insbesondere zur Untersuchung von Aspekten des enterohepatischen Stoffwechsels, d.h. zur Untersuchung des Metabolismus durch Leber-/Darmzellen und anaeroben intestinaler Mikroflora herangezogen. Die Kenntnis des Metabolismus ist für die Entwicklung neuer Pharmaka sowie für die toxikologische bzw. pharmakologische Bewertung von Fremdstoffen von entscheidender Bedeutung.

Chemikalien die in den Organismus gelangen, werden in der Regel durch den Fremstoffmetabolismus in vielfältiger Weise chemisch verändert. Eine wesentliche Aufgabe des Fremdstoffmetabolismus ist es hierbei, lipophile Moleküle in wasserlösliche Umwandlungsprodukte (Metabolite) zu überführen, die dann über die Faeces bzw. den Harn ausgeschieden werden können. Neben dieser entgiftenden Funktion des Fremstoffwechsels kann es aber auch-abhängig von der Chemikalie - zu einer metabolischen Aktivierung (Giftung) durch den Fremstoffmetabolismus kommen. Für das Schicksal eines Fremdstoffes ist insbesondere der enterohepatische Fremdstoffwechsel durch die Leber und den Darm von entscheidender Bedeutung. So treffen oral aufgenommene Substanzen zunächst auf Darm und Leber, die die größte Vielfalt und höchsten Konzentrationen fremdstoffmetabolisierender Enzyme aufweist. Auch die anaerob wachsende intestinale Mikroflora exprimiert zahlreiche fremdstoffmetabolisierende Enzymaktivitäten, wie Glucuronidasen, Sulfatasen, Glucosidasen, Galactosidasen, Acetylasen, Azo- und Nitroreduktasen usw.. Im Rahmen des komplexen Stoffwechsels durch die Leber, den Darm und die intestinalen Mikroflora kann es zu einem Vorgang kommen, der als enterohepatische Zirkulation bezeichnet wird: Fremdstoffe, aber auch endogene Moleküle, werden von der Leberzelle aufgenommen und dort metabolisiert. Werden Konjugate (z.B. mit Sulfat oder Glucuronsäure) gebildet und in die Galle abgegeben so gelangen diese mit der Galle in den Darm. Durch die intestinale Mikroflora des Darms können die Konjugate dekonjugiert werden und die Ausgangssubstanz bei entsprechender Lipidlöslichkeit wieder resorbiert werden und so anschließend wieder in die Leber gelangen, um dort wieder konjugiert zu werden. Der enterohepatische Kreislauf der Substanz kann sich mehrmals wiederholen und führt zu einer deutlichen Verlängerung der Verweildauer der Substanz im Organismus.

Die sequentielle Metabolisierung durch die Leber und die intestinale Mikroflora kann auch zur Aktivierung , d.h. Giftung von Chemikalien führen und spielt daher eine wichtige Rolle bei der Umwandlung von Promutagenen und Prokanzerogenen in ihre ultimalen, reaktiven Metabolite.

Wie oben dargestellt, ist die Kenntnis des enterohepatischen Fremdstoffwechsels für die Entwicklung von Arzneimittelstoffen und von Arzneimitteln sowie für die Bewertung von toxischen Chemikalien von entscheidender Bedeutung. Bisher stehen jedoch keine geeigneten Vorrichtungen bzw. Verfahren zur Verfügung, um den sequentiell ablaufenden Stoffwechsel von Leber/Darm und intestinaler Mikroflora in vitro untersuchen zu können. Eine besondere Schwierigkeit bei der Entwicklung eines entsprechenden In-vitro-Systems zur Untersuchung dieser Stoffwechselvorgänge besteht darin, daß die Zellen, in diesem Fall intestinale Bakterien und Hepatozyten/Darmzellen, auf verschiedene physiologische Umgebungsbedingungen angewiesen sind. So müssen die Säugerzellen unter aeroben, die intestinalen Bakterien jedoch unter anaeroben Bedingungen kultiviert werden. Gleichzeitig müssen die beiden Zellsysteme, d.h. die Hepatozyten/Darmzellen und die intestinalen Bakterien, aber auch in einem engen räumlichen Kontakt stehen, um einen Austausch der Metaboliten bzw. Stoffwechselprodukte zu ermöglichen.

### Stand der Technik

EP-A-0 444 585 offenbart eine Klärvorrichtung mit einem Behälter, der mit einem Wassereinlass, einem Wasserauslass, einem Lufteinlass, einer Luftzuführung und zwei voneinander getrennten Kammerkompartimenten versehen ist. Die mit einer halbdurchlässigen Membran versehenen Kammerkompartimente dienen zur getrennten Kultur von aeroben und anaeroben Bakterien. In dieser Vorrichtung wird nur die aerobe Bakterienkultur (30) mit sauerstoffhaltigem Gas versorgt.

EP-A-0 049 954 offenbart eine Kläreinrichtung, bei der eine gemischte Zellkultur, welche aerobe und anaerobe Mikroorganismen enthält, auf der einen Seite der halbdurchlässigen Membran aufgetragen ist. Ein sauerstoffhaltiger Gasstrom wird durch die andere Seite der Membran der Zellkultur zugeführt.

DE-A- 839 245 offenbart eine Einrichtung zur Züchtung von Mikroorganismen mit einem Behälter. Der Behälter ist durch eine halbdurchlässige Membran in ein unteres und ein oberes Kammerkompartiment geteilt, wobei die (entweder aerobe oder anaerobe) Zellkultur auf der oberen Seite der Membran aufgetragen ist und das Nährmedium in das untere Kammerkompartiment zugeführt wird. Das benötigte Gas zum Wachstum der Mikroorganismen wird nur in das obere Kammerkompartiment zugeführt.

EP-A-0 620 274 offenbart einen Behälter für Zellkultur, der mittels einer halbdurchlässigen Membran in zwei nebeneinander getrennte Kammerkompartimente geteilt ist. Jedes Kompartiment kann Zellen enthalten, wobei die Zellen auf dem Boden des Kompartiments (und nicht auf der Membran) aufgetragen sind. Darüber hinaus ist der Behälter nicht dicht verschlossen, so dass ein freier Gasaustausch zwischen der äußeren Umgebung und dem Inneren des Behälters stattfinden kann.

EP-A-0 725 134 offenbart einen flexiblen Behälter für Zellkultur, der mittels einer halbdurchlässigen Membran in ein oberes und ein unteres Kompartiment geteilt ist, wobei die Zellkultur in das untere Kompartiment und das Nährmedium in das obere Kompartiment zugeführt wird. Die äußeren Wände des Behälters sind aus einem gasdurchlässigen Material hergestellt, so dass ein freier Gasaustausch zwischen den äußeren Umgebung und dem Inneren des Behälters stattfinden kann.

Es ist daher die Aufgabe der vorliegenden Erfindung eine Vorrichtung bzw. ein Verfahren zur Erfassung des Stoffwechsels durch aerobe und anaerobe Zellen in einem In vitro-System zu schaffen.

Diese Aufgabe wird erfindungsgemäß durch eine Vorrichtung mit dem im Patentanspruch 1 angegebenen Merkmalen sowie durch ein Verfahren mit dem im Patentanspruch 13 angegebenen Merkmalen gelöst.

Weitere vorteilhafte Ausgestaltungen der Erfindung sind in den Unteransprüchen angegeben.

Im weiteren wird eine Ausführungsform der Erfindung unter Bezug auf die beigefügten Zeichnungen zur Erklärung der erfindungswesentlichen Eigenschaften beschrieben.

Es zeigen:
- Figur 1: eine schematische Ansicht einer Vorrichtung zur Erfassung des Stoffwechsels von Zellen gemäß der Erfindung;

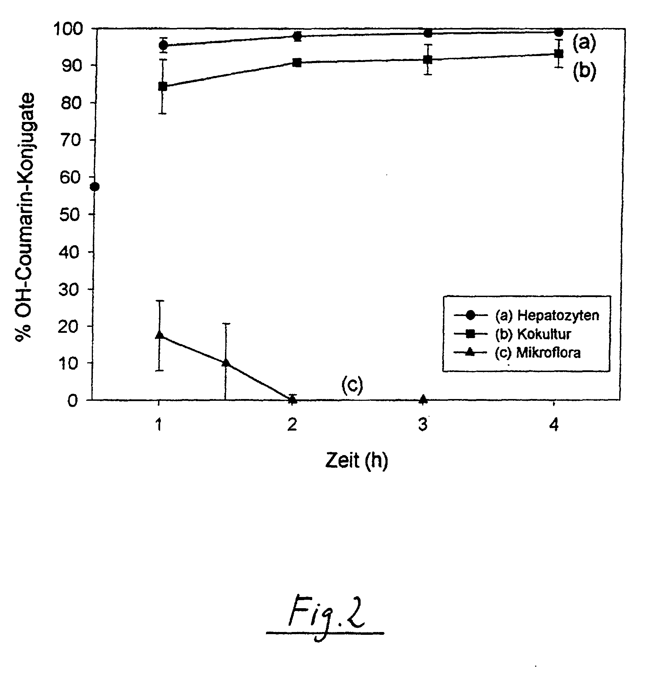

- Figur 2: eine graphische Darstellung von Meßergebnissen, die bei der Untersuchung von Stoffwechselvorgängen von Leberzellen mittels der erfindungsgemäßen Vorrichtung ermittelt wurden.

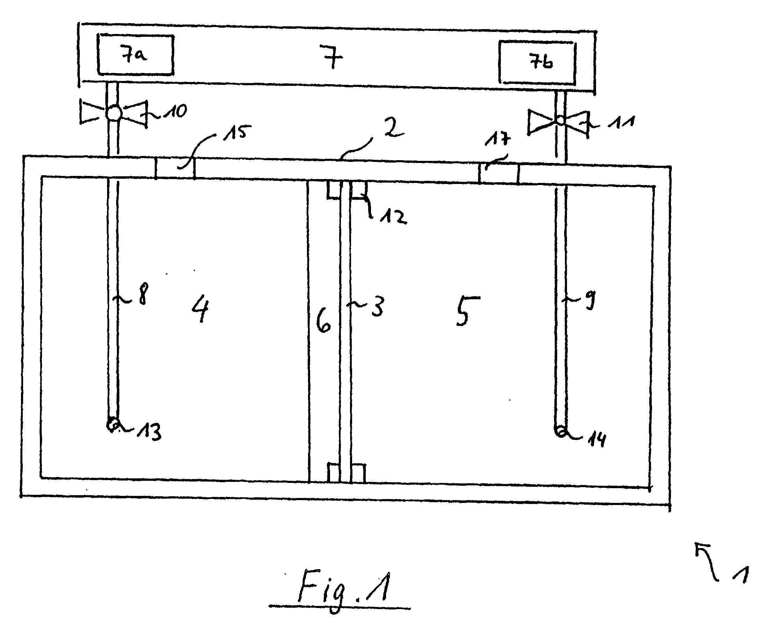

Die Figur 1 zeigt eine Vorrichtung zur Erfassung des Stoffwechsels von Zellen gemäß der Erfindung.

Die Vorrichtung 1 zur Erfassung des Stoffwechsels von Zellen weist eine Kokulturkammer 2 auf, die durch eine permeable Trennwand 3 in ein erstes und zweites Kammerkompartiment 4, 5 getrennt ist. Die permeable, für Zellen undurchlässige Trennwand 3 trägt auf der Seite des ersten Kammerkompartiments 4 aerobe Zellen 6. In dem zweiten Kammerkompartiment 5 befinden sind anaerobe Zellen verteilt.

Die erfindungsgemäße Vorrichtung weist Begasungseinrichtungen 7a, 7b auf, die über ein erstes Gaszufuhrrohr 8 mit dem ersten Kammerkompartiment 4 verbunden ist und über ein zweites Gaszufuhrrohr 9 mit dem zweiten Kammerkompartiment 5 verbunden ist. Die Gaszufuhrrohre 8, 9 weisen jeweils elektronische steuerbare oder handeinstellbare Ventile 10, 11 auf. Die permeable Trennwand 3 wird durch eine Halterung 12 gehalten und ist auswechselbar. Die Gaszufuhrrohre 8, 9 münden in Gaszufuhröffnungen 13, 14 in den entsprechenden Kammerkompartimenten 4, 5. Über das Gaszufuhrrohr 8 wird ein aerobes Gasgemisch in das erste Kammerkompartiment 4 eingeleitet, in dem sich die aeroben Zellen 6 befinden. Zum Schutz von empfindlichen Säugerzellen vor direktem Kontakt mit den Gasblasen befindet sich ein Gitternetz 19 zwischen der Gaszufuhr und den Zellen. In beiden Kammerkompartimenten befinden sind Kulturmedien, die jeweils eine geeignete Umgebung für die aeroben bzw. anaeroben Zellen schaffen. Die Begasung des Kulturmediums für die aeroben Zellen 6 in dem ersten Kammerkompartiment 4 von unten über die Gaseinlaßöffnung 13 führt zu einer Durchmischung des Kulturmediums in dem Kammerkompartiment 4. Das in das erste Kammerkompartiment 4 zugeführte Gasgemisch durchströmt das Kulturmedium von unten nach oben und kann durch eine Gasaustrittsöffnung 15 entweichen. Das zugeführte aerobe Gasgemisch ist beispielsweise Druckluft mit einem Anteil von 10 % CO₂. Handelt es sich bei den aeroben Zellen 6 um Hepatozyten, wird vorzugsweise ein aerobes Gasgemisch von 19 % O₂, 71 % N₂ und 10 % CO₂ bzw. eine Druckluft mit 10 % CO₂ zugeführt. Die Gasaustrittsöffnung 15 des Kammerkompartiments 4, wird gleichzeitig als eine Entnahme- bzw. Zuführöffnung verwendet, durch die das Kulturmedium in das Kammerkompartiment 4 eingeführt bzw. aus ihr entnommen werden kann. Zusätzlich dient diese Gasaustrittsöffnung 15 dazu, Proben aus dem Kammerkompartiment 4 zu Analysezwecken zu entnehmen.

Über das Gaszufuhrrohr 9 und die Einlaßöffnung 14 wird ein anaerobes Gasgemisch in das zweite Kammerkompartiment 5, in dem sich die anaeroben Zellen bzw. anaeroben Bakterien befinden, zugeführt. Das zweite Kammerkompartiment 5 ist ebenfalls mit einem Kulturmedium gefüllt, welches für die anaeroben Bakterien geeignete Lebensbedingungen schafft. Das zweite Kammerkompartiment 5 weist eine Austrittsöffnung 17 auf, aus der das zugeführte Gas nach Durchmischung mit dem Kulturmedium entweichen kann. Die Öffnung 17 wird gleichzeitig zur Zuführung bzw. Entnahme des Kulturmediums. Über die Austrittsöffnung 17 können ebenfalls Proben aus dem zweiten Kammerkompartiment 5 zu Analysezwecken entnommen werden. Das über die Gaszufuhrleitung 9 zugeführte anaerobe Gasgemisch besteht beispielsweise aus 90 % N₂ und 10 % CO₂.

Die beiden zugeführten Gasgemische weisen vorzugsweise einen Anteil von 10% CO₂ auf. Dieser Anteil von CO₂ in den zugeführten Gasen dient als eine Puffer-Komponente, deren Gegenspieler eine Konzentration von 3,7 g NaCO₃ in dem Kulturmedium ist. Auf diese Weise kann das Kulturmedium entsprechend dem Anteil der Pufferkomponenten auf einen gewünschten pH-Wert, in diesem Fall auf einen pH-Wert von 7,4 eingestellt werden. Die pH-Regulation der beiden Kulturmedien ist also jeweils durch die Zusammensetzung der zugeführten anaeroben und aeroben Gasgemische in Kombination mit dem NaCO₃-Gehalt des Mediums einstellbar.

Bei der permeablen Trennwand 3 handelt es sich vorzugsweise um eine poröse Membran. Die Membran erlaubt den Durchtritt von Metaboliten von einem Kammerkompartiment in das andere Kammerkompartiment. Gleichzeitig dient die Membran als Kultivierungsunterlage für die aerob wachsenden Zellen, z.B. für Hepatozyten. Die Membran weist beispielsweise eine Porengröße von 0,4 µm auf und kann als Kultivierungsunterlage für beliebige Säugerzellen dienen. Die permeable Membran 3 kann aus einem beliebigen Membranmaterial bestehen, das in Abhängigkeit der zu untersuchenden Zellen auswählbar ist. Die permeable Membran 3 erlaubt den Durchtritt von Metaboliten (Stoffwechselprodukten), ist jedoch für anaerobe Bakterien, die sich in dem zweiten Kammerkompartiment 5 befinden, und für die aeroben Säugerzellen undurchlässig. Die Membran 3 kann beispielsweise aus Polycarbonat bestehen.

Bei einer weiteren Ausführungsform wird die permeable Trennwand 3 durch ein Gewebeisolat, wie beispielsweise Darmgewebeisolat, gebildet.

Die Trennwandung 3 ist in die Halterung 12 einspannbar und kann nach jedem Bestimmungsvorgang ausgewechselt werden. Zur Erleichterung des Auswechselns der Trennwand 3 ist die Kulturkammer 2 bei einer bevorzugten Ausführungsform aufklappbar gestaltet. Bei einer weiteren Ausführungsform der erfindungsgemäßen Vorrichtung 1 kann die permeable Trennwand seitlich in die Kokulturkammer 2 eingeschoben werden.

Bei einer bevorzugten Ausführungsform ist die Kulturoberfläche für die zu untersuchenden Zellen relativ groß, während das Volumen der Kompartiments möglichst klein gewählt wird. Je größer das Verhältnis der Kulturoberfläche zu dem Volumen des Kompartiments 4, 5 ist, desto höher sind die Aufkonzentrierungen von Metaboliten in dem jeweiligen Kulturmedium und desto einfacher gestaltet sich ein anschließender analytischer Nachweis. Bei einer bevorzugten Ausführungsform beträgt die Kulturoberfläche für die Zellen 20 cm² und das Volumen je Kompartiment 7 ml.

Bei einer weiteren Ausführungsform steht die Vorrichtung 1 in einem Inkubator, welcher die Kokulturkammer 2 temperiert, beispielsweise auf 37°C. Der nicht gezeigte Inkubator weist Öffnungen auf, aus denen das aus den Kammerkompartiments 4, 5 über die Öffnungen 15, 17 austretende Gas entweichen kann.

Bei einer weiteren bevorzugten Ausführungsform werden mehrere Kokulturkammern 2 parallel geschaltet und durch eine Begasungseinrichtung 7 gleichzeitig parallel mit Gas versorgt. Dabei kann der Gasfluß über einen Verteilerkamm geregelt werden. Um eine gleichmäßige Begasung der einzelnen Kokulturkammern 2 in gewünschter Stärke zu erhalten, wird der Gasfluß zu den verschiedenen Kokulturkammern 2 über Ventile manuell oder elektronisch eingestellt.

Die Kokulturkammern 2 bestehen bei einer bevorzugten Ausführungsform aus einem durchsichtigen Material, beispielsweise Plexiglas.

Im weiteren wird das erfindungsgemäße Verfahren zur Erfassung des Stoffwechsels von Zellen beispielhaft anhand der Erfassung des Stoffwechsels durch Leberzellen und intestinalen Bakterien des Menschen beschrieben.

Die Kokulturkammer 2 wird zunächst mit Barrycidal antimikrobiell gespült und drei Stunden bei 65 - 70°C in einem Brutschrank sterilisiert. Die Halterungen 12 werden mit eingespannter Trennwand 3 in 70% EtOH sterilisiert. Als Trennwand wird eine Membran aus Polycarbonat mit einer Porengröße von 0,4 µm bei einem Durchmesser von 7 cm verwendet. Ein William's E Medium wird auf 3,7 g Bicarbonat/ 1000 ml eingestellt. Es werden frisch isolierte Hepatozyten und Faeces-Suspensions-Kryokulturen verwendet.

Die sterile, in die Halterung 12 eingespannte Membran 3 wird mit 0,2 mg Kollagen/cm² beschichtet. Auf die vorbeschichtete Membran 3 werden 1,2 x 10⁵/cm² frisch isolierte Rattenhepatozyten in dem Kulturmedium ausgebreitet und zur Anheftung an die Membran eine Stunde in einem Brutschrank kultiviert. Dabei heften sich etwa 90% der Hepatozyten an die Membran 3 an. Die Membran wird einschließlich ihrer Halterung 12 vom Brutschrank in die Kokulturkammer 2 überführt. Die Kammerkompartimente 4 und 5 werden mit einem vorgewärmten Kulturmedium gefüllt und die Gaszufuhr über die Gaseinlaßventile 10, 11 eingestellt. Die Gaseinlaßventile 10, 11 werden so geregelt, daß die Begasung eines Kompartiments 4, 5 etwa 8 ml/Stunde beträgt. Das Kammerkompartiment 4, in dem sich die Hepatozyten befinden, wird mit 19 % O₂, 71 % N₂ und 10 % CO₂, was einer Druckluft mit 10 % CO₂ entspricht, begast. Das Kammerkompartiment 5, das durch die Trennwand 3 von den Hepatozyten getrennt ist, wird mit 90% N₂ und 10 % CO₂ begast. Nach etwa 15 Minuten wird in das anaerobe Kammerkompartiment 5 eine definierte Probe einer frisch aufgetauten humanen Faeces-Kryokultur pipettiert (Endkonzentration: 0,01 g Faeces/ml). Die Kokultur kann nun zur Untersuchung der Biotransformation von Substanzen eingesetzt werden. Die Kultivierungszeit beträgt vorzugsweise nicht mehr als 4 Stunden.

Es wurden Vorversuche zum Durchtritt von Produkten der Leberzellen durch die permeable Membran durchgeführt. Als Modellsubstanz wurde beispielsweise der Durchtritt der Lactat-Dehydrogenase (LDH) untersucht. Lactat-Dehydrogenase ist ein relativ großes Protein, das durch die Hepatozyten während ihrer Kultivierung freigesetzt wird. Die Verteilung der Lactat-Dehydrogenase beträgt etwa 60 % in dem ersten Kammerkompartiment 4 und etwa 40 % in dem Kammerkompartiment 5, in welchem sich die anaeroben Bakterien befinden.

Weiterhin konnte festgestellt werden, daß sich die Vitalität der Hepatozyten durch die Anwesenheit der anaeroben Bakterien in dem Kammerkompartiment 5 nicht mindert. Es wurden hierzu die Aktivitäten wichtiger fremdstoffmetabolisierender Enzyme der Hepatozyten untersucht. Aktivitäten wie die Ethoxycoumarin-Deethylase und der Testosteron-Hydroxylierung werden durch eine Kokultivierung der Hepatozyten mit den intestinalen Bakterien innerhalb einer Kultivierungszeit von 4 Stunden nicht verändert.

Als Modellsubstrat wurde Ethoxycoumarin wurde zur Untersuchung des Zusammenspiels des Metabolismus der Hepatozyten und der intestinalen anaeroben Bakterien in die Kokultur eingesetzt.

Es ist bekannt, daß die Hepatozyten das Substrat Ethoxycoumarin zur Hydroxyverbindung (OH-Coumarin) deethylieren und anschließend konjugieren. Durch die intestinalen anaeroben Bakterien, die sich in dem zweiten Kammerkompartiment 5 befinden, können die durch die Hepatozyten gebildeten Konjugate gespalten werden, d.h. wieder in das meßbare OH-Coumarin überführt werden.

Um die oben dargestellten metabolischen Umsetzungen in dem Kokultivierungssystem zu erfassen, wurden drei verschiedene Versuche durchgeführt: Inkubation von
(a) Ethoxycoumarin mit Hepatozyten,
(b) Ethoxycoumarin mit Hepatozyten und intestinaler Mikroflora und
(c) Inkubation von bereits gebildeten Konjugaten des OH-Coumarin mit intestinaler Mikroflora.

Bei der Inkubationen des Ethoxycoumarin nur mit Hepatozyten, wurde die Testsubstanz wie erwartet fast vollständig zu OH-Coumarin-Konjugaten umgesetzt. Wurden intestinale Mikroflora und OH-Coumarin-Konjugate in das Kultivierungssytem eingesetzt, so lag nach einer Stunde nur noch etwa 20% Konjugat vor und nach zwei Stunden waren keine Konjugate mehr nachzuweisen. Dies spricht für die effiziente Spaltung der Konjugate durch die Mikroflora. Wurde das Ethoxycoumarin in Gegenwart von Hepatozyten und intestinaler Mikroflora inkubiert, liegen nur etwa 90% als Konjugat des OH-Coumarin vor. Das Ergebnis dieser Kokultivierung läßt darauf schließen, daß die von den Hepatozyten gebildeten Konjugate durch die intestinale Mikroflora wieder gespalten wurden. Es liegt aber dennoch ein großer Anteil des Substrats in konjugierter Form vor, da die Hepatozyten die Spaltprodukte der intestinalen Mikroflora wieder aufnehmen und erneut konjugieren. Es ist demnach also anzunehmen, daß die Substanz Ethoxycoumarin während des Metabolismus in unserem Invitro-System einen Kreislauf zwischen Leberzellen und intestinaler Mikroflora durchläuft.

Das oben beschriebene Versuchsbeispiel zeigt, daß die erfindungsgemäße Vorrichtung bzw. das erfindungsgemäße Verfahren zur Erfassung von komplexen Stoffwechselvorgängen bei Zellen geeignet ist. Bei den aeroben Zellen kann es sich beispielsweise auch um Darmzellen handeln.

Die erfindungsgemäße Vorrichtung eignet sich zur Untersuchung von Stoffwechselvorgängen beliebiger Zellen und vorzugsweise zur Untersuchung von Stoffwechselvorgängen, bei denen Zellen, die in einer ersten Umgebung existieren, mit anderen Zellen zusammenwirken, die in einer anderen Umgebung existieren, wobei die Zellen gleichzeitig in einem engen räumlichen Kontakt stehen müssen.

## Patentansprüche

1. Vorrichtung zur Erfassung der Interaktionen des Stoffwechsels von aeroben und anaeroben Zellsystemen mit mindestens einer Kokulturkammer (2), die durch eine permeable, für Zellen undurchlässige Trennwandung (3) in ein erstes und zweites Kammerkompartiment (4,5) getrennt ist,
wobei die permeable Trennwandung (3) auf der Seite des ersten Kammerkompartiments (4) aerobe Zellen trägt und sich in dem zweiten Kammerkompartiment (5) anaerobe Zellen befinden, und mit Begasungseinrichtungen (7a, 7b), die das erste Kammerkompartiment (4) mit einem aeroben Gasgemisch und das zweite Kammerkompartiment (5) mit einem anaeroben Gasgemisch versorgt,
wobei die Zellen jeweils Stoffwechselprodukte erzeugen, die durch die permeable Trennwandung (3) in das jeweils andere Kammerkompartiment hindurchtreten können und jeweils analysierbar sind.

2. Vorrichtung nach Anspruch 1,
**dadurch gekennzeichnet,**
**daß** die permeable Trennwand (3) eine permeable Membran ist.

3. Vorrichtung nach Anspruch 1 oder 2,
**dadurch gekennzeichnet,**
**daß** die permeable Membran aus Polycarbonat besteht oder ein Gewebeisolat ist.

4. Vorrichtung nach einem der Ansprüche 1 bis 3,
**dadurch gekennzeichnet,**
**daß** die Membran eine Porengröße von 0,2 - 4,0 µm aufweist.

5. Vorrichtung nach einem der vorhergehenden Ansprüche,
**dadurch gekennzeichnet,**
**daß** die Kokulturkammer (2) temperierbar ist.

6. Vorrichtung nach einem der vorhergehenden Ansprüche,
**dadurch gekennzeichnet,**
**daß** sich in den Kammerkompartiments (4,5) jeweils ein Kulturmedium befindet, dessen pH-Wert einstellbar ist.

7. Vorrichtung nach einem der vorhergehenden Ansprüche,
**dadurch gekennzeichnet,**
**daß** der pH-Wert des Kulturmediums durch die Zusammensetzung der durch die Begasungseinrichtungen (7a, 7b) abgegebenen Gasgemische einstellbar ist.

8. Vorrichtung nach einem der vorhergehenden Ansprüche,
**dadurch gekennzeichnet,**
**daß** die Zufuhr der Gasgemische zu den beiden Kammerkompartiments (4,5) über Ventile (10,11) manuell oder elektronisch einstellbar ist.

9. Vorrichtung nach einem der vorhergehenden Ansprüche,
**dadurch gekennzeichnet,**
**daß** die aeroben Zellen Säugetierzellen sind.

10. Vorrichtung nach Anspruch 9,
**dadurch gekennzeichnet,**
**daß** die Säugetierzellen Leberzellen sind.

11. Vorrichtung nach einem der vorhergehenden Ansprüche,
**dadurch gekennzeichnet,**
**daß** die anaeroben Zellen anaerobe Bakterien, bevorzugt der intestinalen Mikroflora, sind.

12. Vorrichtung nach einem der vorhergehenden Ansprüche,
**dadurch gekennzeichnet,**
**daß** mehrere Kokulturkammern parallel angeordnet sind.

13. Verfahren zur Erfassung der Interaktionen des Stoffwechsels von aeroben und anaeroben Zellsystemen in vitro mit den folgenden Schritten:
Aufbringen erster Zellen (6) auf die zum ersten Kammerkompartiment (4) zugeordneten Seite einer für die Stoffwechselprodukte der Zellen permeablen, für Zellen undurchlässige Trennwandung (3), die eine Kokulturkammer (2) in ein erstes und ein zweites je ein Kulturmedium enthaltendes Kammerkompartiment (4,5) trennt,
Zuführen eines aeroben Gasgemisches in das erste Kammerkompartiment (4) und eines anaeroben Gasgemisches in das zweite Kammerkompartiment (5),
Einführen zweiter Zellen in das zweite Kammerkompartiment (5), und
Entnehmen von Proben aus den Kammerkompartiments (4,5) nach einer Inkubationszeit zu deren Analyse.

14. Verfahren nach Anspruch 12,
**dadurch gekennzeichnet,**
**daß** die permeable Trennwandung vor jedem Experiment ausgewechselt wird.

15. Verfahren nach einem der vorhergehenden Ansprüche,
**dadurch gekennzeichnet,**
**daß** die Proben über Pipetten automatisch entnommen werden und einer Analysevorrichtung zugeführt werden.

16. Verfahren nach einem der vorhergehenden Ansprüche,
**dadurch gekennzeichnet,**
**daß** die entstandenen Stoffwechselprodukte bezüglich ihrer Konzentration und chemischen Zusammensetzung analysiert werden.

17. Verfahren nach einem der vorhergehenden Ansprüche,
**dadurch gekennzeichnet,**
**daß** die Gasgemischzufuhr in ihrer Zusammensetzung und Menge automatisch geregelt wird.

18. Verfahren nach einem der vorhergehenden Ansprüche,
**dadurch gekennzeichnet,**
**daß** als erste Zellen Leberzellen und als zweite Zellen Komponente anaerobe Bakterien, bevorzugt der intestinalen Mikroflora, eingesetzt werden.

19. Verfahren nach einem der vorhergehenden Ansprüche,
**dadurch gekennzeichnet,**
**daß** das Verfahren zur Erfassung von Aspekten des enterohepatischen Stoffwechsels eingesetzt wird.

20. Verfahren nach einem der vorhergehenden Ansprüche,
**dadurch gekennzeichnet,**
**daß** in das erste Kammerkompartiment und/oder in das zweite Kammerkompartiment Chemikalien eingeführt werden, deren Verstoffwechselung durch die ersten Zellen und/oder die zweiten Zellen analysiert werden soll.

21. Verfahren nach einem der vorhergehenden Ansprüche,
**dadurch gekennzeichnet,**
**daß** das Gasgemisch je in den unteren Abschnitt der Kammerkompartimente derart eingeführt wird, daß eine Durchmischung der Kulturmedien in den Kammerkompartimenten stattfindet.

22. Verwendung der Vorrichtung nach Anspruch 1 zur Erfasssung des Stoffwechsels von Säugetierzellen, insbesondere von Leberzellen und/oder von Bakterien der intestinalen Mikroflora.

## Claims

1. A device of the measurement of the interactions of the metabolism of aerobic and anaerobic cell systems comprising at least one co-culturing chamber (2) which is separated by a permeable, cell-impermeable separating wall (3) into a first and a second chamber compartment (4, 5),
wherein the permeable separating wall (3) carries aerobic cells on the side facing the first chamber compartment (4) and wherein anaerobic cells are present in the second chamber compartment (5); and comprising gas supply means (7a, 7b) supplying the first chamber compartment (4) with an aerobic gas mixture and the second chamber compartment (5) with an anaerobic gas mixture;
wherein each of the cells generates metabolic products which can pass through the permeable separating wall (3) into the respective other chamber compartment and can be analyzed.

2. A device according to claim 1 **characterized in that** the permeable separating wall (3) is a permeable membrane.

3. A device according to claim 1 or 2 **characterized in that** the permeable membrane is made of polycarbonate or is a tissue isolate.

4. A device according to any of claims 1 to 3 **characterized in that** the membrane has a pore size of 0.2-0.4 µm.

5. A device according to any of the preceding claims **characterized in that** the temperature in the co-culturing chamber (2) may be thermostatically controlled.

6. A device according to any of the preceding claims **characterized in that** each of the chamber compartments (4, 5) contains a culture medium the pH value of which may be adjusted.

7. A device according to any of the preceding claims **characterized in that** the pH value of the culture medium may be adjusted by means of the composition of the gas mixtures delivered by the gas supply means (7a, 7b).

8. A device according to any of the preceding claims **characterized in that** feeding of the gas mixtures to the two chamber compartments (4, 5) may be controlled via valves (10, 11) in a manual or electronic manner.

9. A device according to any of the preceding claims **characterized in that** the aerobic cells are mammalian cells.

10. A device according to claim 9 **characterized in that** the mammalian cells are liver cells.

11. A device according to any of the preceding claims **characterized in that** the anaerobic cells are anaerobic bacteria, preferably of the intestinal microflora.

12. A device according to any of the preceding claims **characterized in that** a plurality of co-culturing chambers is arranged in parallel.

13. A method for the measurement of the interactions of the metabolism of aerobic and anaerobic cell systems in vitro comprising the following steps of:
applying first cells (6) to the side facing the first chamber compartment (4) of a permeable, cell-impermeable separating wall (3) separating a co-culturing chamber (2) into a first and a second chamber compartment (4, 5) each containing a culture medium;
feeding an aerobic gas mixture into the first chamber compartment (4) and an anaerobic gas mixture into the second chamber compartment (5);
introducing second cells into the second chamber compartment (5); und
removing samples form the chamber compartments (4, 5) for the analysis thereof after an incubation period.

14. A method according to claim 12 **characterized in that** the permeable separating wall is replaced prior to each experiment.

15. A method according to any of the preceding claims **characterized in that** the samples are removed automatically via pipettes and are delivered to an analytical device.

16. A method according to any of the preceding claims **characterized in that** the metabolic products generated are analyzed with respect to their concentration and chemical composition.

17. A method according to any of the preceding claims **characterized in that** the gas mixture feed is controlled with respect to its composition and amount.

18. A method according to any of the preceding claims **characterized in that** liver cells are used as said first cells and anaerobic bacteria, preferably of the intestinal microflora are used as said second cells.

19. A method according to any of the preceding claims **characterized in that** said method is used for the measurement of aspects of the enterohepatic metabolism.

20. A method according to any of the preceding claims **characterized in that** into said first chamber compartment and/or said second chamber compartment chemicals are introduced the metabolism of which by said first cells and/or by said second cells is to be analyzed.

21. A method according to any of the preceding claims **characterized in that** each of said gas mixtures is introduced into the bottom part of said chamber compartments in a manner to achieve thorough mixing of the culture media in the chamber compartments.

22. The use of the device according to claim 1 for the measurement of the metabolism of mammalian cells, particularly of liver cells, and/or bacteria of the intestinal microflora.

## Revendications

1. Dispositif pour la détection des interactions du métabolisme de systèmes de cellules aérobies et anaérobies, comprenant au moins une chambre de co-culture (2), qui est divisée en un premier et un second compartiment par une cloison perméable (3) imperméable aux cellules, dans lequel la cloison perméable (3) porte des cellules aérobies sur le côté du premier compartiment (4) de la chambre, tandis qu'il y a des cellules anaérobies dans le second compartiment (5) de la chambre, et des dispositifs de gazéification (7a, 7b), qui alimentent le premier compartiment (4) de la chambre en mélange gazeux aérobie et le second compartiment (5) de la chambre en mélange gazeux anaérobie, les cellules produisant respectivement des produits métaboliques, qui peuvent pénétrer, à travers la cloison perméable (3), dans l'autre compartiment respectif de la chambre et être respectivement analysés.

2. Dispositif selon la revendication 1, **caractérisé en ce que** la cloison perméable (3) est une membrane perméable.

3. Dispositif selon la revendication 1 ou 2, **caractérisé en ce que** la membrane perméable est constituée d'un polycarbonate ou est un isolat de tissu.

4. Dispositif selon l'une quelconque des revendications 1 à 3, **caractérisé en ce que** la membrane a une taille de pores de 0,2 à 4,0 µm.

5. Dispositif selon l'une quelconque des revendications précédentes, **caractérisé en ce que** la chambre de co-culture (2) peut être tempérée.

6. Dispositif selon l'une quelconque des revendications précédentes, **caractérisé en ce qu'**il y a dans les compartiments (4,5) de la chambre respectivement un milieu de culture dont le pH peut être réglé.

7. Dispositif selon l'une quelconque des revendications précédentes, **caractérisé en ce que** le pH du milieu de culture peut être réglé par la composition des mélanges gazeux délivrés par les dispositifs de gazéification (7a, 7b).

8. Dispositif selon l'une quelconque des revendications précédentes, **caractérisé en ce que** l'acheminement des mélanges gazeux aux deux compartiments (4, 5) de la chambre peut être réglé manuellement ou électroniquement par des soupapes (10, 11).

9. Dispositif selon l'une quelconque des revendications précédentes, **caractérisé en ce que** les cellules aérobies sont des cellules de mammifères.

10. Dispositif selon la revendication 9, **caractérisé en ce que** les cellules de mammifères sont des cellules de foie.

11. Dispositif selon l'une quelconque des revendications précédentes, **caractérisé en ce que** les cellules anaérobies sont des bactéries anaérobies, de préférence de la microflore intestinale.

12. Dispositif selon l'une quelconque des revendications précédentes, **caractérisé en ce que** plusieurs chambres de co-culture sont aménagées en parallèle.

13. Procédé pour détecter les interactions du métabolisme de systèmes de cellules aérobies et anaérobies in vitro, comprenant les étapes suivantes :
application de premières cellules (6) sur le côté, tourné vers le premier compartiment (4) de la chambre, d'une cloison (3) perméable aux produits métaboliques des cellules et imperméable aux cellules, qui divise une chambre de co-culture (2) en premier et second compartiments (4, 5) contenant respectivement un milieu de culture;
acheminement d'un mélange gazeux aérobie dans le premier compartiment (4) de la chambre et d'un mélange gazeux anaérobie dans le second compartiment (5) de la chambre;
introduction de secondes cellules dans le second compartiment (5) de la chambre; et
prélèvement d'échantillons des compartiments (4, 5) de la chambre après une période d'incubation pour leur analyse.

14. Procédé selon la revendication 12, **caractérisé en ce que** la cloison perméable est remplacée avant chaque expérimentation.

15. Procédé selon l'une quelconque des revendications précédentes, **caractérisé en ce que** les échantillons sont prélevés automatiquement à l'aide de pipettes et acheminés à un dispositif d'analyse.

16. Procédé selon l'une quelconque des revendications précédentes, **caractérisé en ce que** les produits métaboliques obtenus sont analysés en termes de concentration et de composition chimique.

17. Procédé selon l'une quelconque des revendications précédentes, **caractérisé en ce que** l'acheminement de mélanges gazeux est régulé automatiquement en termes de composition et de quantité.

18. Procédé selon l'une quelconque des revendications précédentes, **caractérisé en ce qu'**on utilise comme premières cellules des cellules de foie et comme secondes cellules des bactéries anaérobies, de préférence de la microflore intestinale.

19. Procédé selon l'une quelconque des revendications précédentes, **caractérisé en ce qu'**on utilise le procédé pour saisir des aspects du métabolisme entérohépatique.

20. Procédé selon l'une quelconque des revendications précédentes, **caractérisé en ce qu'**on introduit dans le premier compartiment et/ou dans le second compartiment de la chambre des produits chimiques dont le métabolisme doit être analysé par les premières cellules et/ou les secondes cellules.

21. Dispositif selon l'une quelconque des revendications précédentes, **caractérisé en ce que** le mélange gazeux est introduit respectivement dans la section inférieure des compartiments de la chambre de sorte qu'il se produise un mélange des milieux de culture dans les compartiments de la chambre.

22. Utilisation du dispositif selon la revendication 1 pour la détection du métabolisme de cellules de mammifères, en particulier de cellules du foie et/ou de bactéries de la microflore intestinale.
